# EUROPÄISCHE PATENTSCHRIFT

(11) **EP 1 699 480 B1**
(45) Veröffentlichungstag und Bekanntmachung des Hinweises auf die Patenterteilung: **15.06.2011**
(21) Anmeldenummer: 04728317.1
(22) Anmeldetag: 20.04.2004
(51) Int. Cl.: A61K 39/00, C12N 5/09, C12N 5/10, A61P 35/00

(54) **ALLOGENES TUMORTHERAPEUTIKUM**
ALLOGENEIC TUMOR THERAPEUTIC AGENT
AGENT THERAPEUTIQUE ANTITUMORAL ALLOGENE

(30) Priorität: 30.12.2003 WO PCT/DE03/04299
(43) Veröffentlichungstag der Anmeldung: 13.09.2006
(73) Patentinhaber: Mologen AG, 14195 Berlin (DE)
(72) Erfinder: DOBRIC, Tomislav, 14532 Kleinmachnow (DE); WITTIG, Burghardt, 14129 Berlin (DE); SCHMIDT, Manuel, 14195 Berlin (DE)
(74) Vertreter: Harrison, Robert John
(86) Internationale Anmeldenummer: PCT/DE2004/000859
(87) Internationale Veröffentlichungsnummer: WO 2005/063280

(56) Entgegenhaltungen:
- WO-A-93/06867
- WO-A-02/060476
- WO-A-03/045428
- US-A- 6 039 941
- SCHAKOWSKI F ET AL: "A NOVEL MINIMAL-SIZE VECTOR (MIDGE) IMPROVES TRANSGENE EXPRESSION IN COLON CARCINOMA CELLS AND AVOIDS TRANSFECTION OF UNDESIRED DNA" MOLECULAR THERAPY, ACADEMIC PRESS, SAN DIEGO, CA,, US, Bd. 3, Nr. 5, PART 1, Mai 2001 (2001-05), Seiten 793-800, XP001100620 ISSN: 1525-0016
- KÖCHLING JOACHIM ET AL: "Protection of mice against Philadelphia chromosome-positive acute lymphoblastic leukemia by cell-based vaccination using nonviral, minimalistic expression vectors and immunomodulatory oligonucleotides." CLINICAL CANCER RESEARCH : AN OFFICIAL JOURNAL OF THE AMERICAN ASSOCIATION FOR CANCER RESEARCH. 1 AUG 2003, Bd. 9, Nr. 8, 1. August 2003 (2003-08-01), Seiten 3142-3149, XP002308451 ISSN: 1078-0432
- VEREECQUE R ET AL: "Gene transfer of GM-CSF, CD80 and CD154 cDNA enhances survival in a murine model of acute leukemia with persistence of a minimal residual disease." GENE THERAPY. AUG 2000, Bd. 7, Nr. 15, August 2000 (2000-08), Seiten 1312-1316, XP002308255 ISSN: 0969-7128
- NOGUCHI M ET AL: "Induction of antitumor immunity by transduction of CD40 ligand gene and interferon-gamma gene into lung cancer." CANCER GENE THERAPY. JUN 2001, Bd. 8, Nr. 6, Juni 2001 (2001-06), Seiten 421-429, XP002308452 ISSN: 0929-1903
- CHIODONI C ET AL: "Dendritic cells infiltrating tumors cotransduced with granulocyte/macrophage colony-stimulating factor (GM-CSF) and CD40 ligand genes take up and present endogenous tumor-associated antigens, and prime naive mice for a cytotoxic T lymphocyte response." THE JOURNAL OF EXPERIMENTAL MEDICINE. 5 JUL 1999, Bd. 190, Nr. 1, 5. Juli 1999 (1999-07-05), Seiten 125-133, XP002308453 ISSN: 0022-1007

## Beschreibung

Die Erfindung betrifft eine Vakzine auf der Basis allogener Tumorzellen zur therapeutischen Behandlung von Tumorerkrankungen, sowie ein Verfahren zur Herstellung einer solcher Vakzine; ferner transfizierte humane Tumorzellen zur Verwendung als Vakzine.

Neben den konventionellen Behandlungsmethoden von Krebserkrankungen, wie der Strahlen - u. Chemotherapie, die seit den 1950er Jahren die einzige Behandlungsoption für fortgeschrittene und weitgestreute Tumorerkrankungen darstellt, scheint die Immuntherapie bei metastasierenden Tumoren ein neuer erfolgsversprechender Ansatz zu sein.

Der Immuntherapie zielt darauf ab, die natürliche Immunantwort gegen die Tumorerkrankung durch gentechnologische Modifikationen zu verstärken, also die "Aufmerksamkeit" des Immunsystems gegenüber Krebszellen und damit die Abwehrreaktion so zu beeinflussen, daß der Tumor vom Körper selbst bekämpft wird.

Da einige maligne Erkrankungen wie z.B. das fortgeschrittene Nierenzellkarzinom relativ sensitiv für immuntherapeutische Ansätze zu sein scheinen, jedoch die systemische Therapie mit Zytokinen wie IL-2 und IFN-alpha mit z.T. erheblichen Nebenwirkungen einhergeht, wurden andere immuntherapeutische Protokolle entwickelt.

Die meisten klinischen Studien setzen derzeit auf die Entfernung des Tumors, einer anschließenden ex-vivo Transfektion der Tumorzellen mit einem therapeutischen Gen, Bestrahlung der Tumorzell-Population und nachfolgender Reimplantation der nunmehr modifizierten Tumorzellen. Durch diese Tumorzellvakzinierung läßt sich die Antitumorantwort in Abhängigkeit vom transfizierten therapeutischen Gen in unterschiedlichem Ausmaße steigern.

Basierend auf diesem Ansatz sowie tierexperimentellen Ergebnissen wurde eine Reihe von klinischen Studien der Phase I und II genehmigt (Finke et al., 1997; Wittig et al., 2001).

Erste Ergebnisse mit transfizierten singulären therapeutischen Genen ergaben allerdings neben einer guten Tolerabilität der Therapie nur in wenigen Fällen partielle oder komplette Remissionen.

In einem Kolonkarzinom-Mausmodell konnte der Gentransfer von CD40UCD154 eine Ausschüttung von Zytokinen, eine Eradikation des Tumors und eine Immunität erzeugen (Sun et al., 2000).

Die Erfinder der vorliegenden Anmeldung konnten zeigen, dass der Transfer von Expressionsplasmiden für humanes Interleukin 7 (IL-7) in Tumorzellen zu einer erhöhten Sensitivität gegen Effektorzellen des Immunsystems führt, besonders bei autologem Transfer (Finke et al., 1997, Cancer Gene Ther. 4: 260-268). Ebenso konnten die Erfinder zeigen, das nach Transfektion zweier therapeutischer Gene (IL-7, GM-CSF) in autologe Tumorzellen, bei der Hälfte der behandelten Patienten eine klinisch signifikante Reaktion zu verzeichen war (WO 02/060476).

Aus der US 5,681,562 ist bekannt, Patienten mit bestimmten Krebserkrankungen Zellen zu injizieren, die mit für Zytokine kodierender DNA oder RNA transfiziert wurden sind. Das Immunsystem des Patienten soll so gegen Antigene von Tumoren stimuliert werden. In der Patentschrift werden Versuche beschrieben, in denen Fibroblasten von Mäusen durch retrovirale Vektoren, kodierend für IL-2 transfiziert wurden. Ebenso beschrieben ist der in-vivo Versuch, in dem die Wirksamkeit der Behandlung an einem murinen Dickdarmkarzinom-Modell getestet wurde. Die Mäuse, denen transfizierte Fibroblasten s.c. injiziert wurden, entwickelten im Vergleich zu Kontrollgruppen ein deutlich verlangsamtes Tumorwachstum. In in-vitro Versuchen mit humanen transfizierten Fibroblasten konnte ebenfalls ein deutlich erhöhtes IL-2 Expressionsniveau bestimmt werden. Neben fehlenden klinischen Daten, die über das Maus-Modell hinausgehen, sind die als Expressionsvektor verwendeten viralen Vektoren als wenig vorteilhaft anzusehen. Durch die Instabilität des attenuierten Impfstammes ist eine Rückverwandlung in einen virulenten Stamm nicht auszuschließen, außerdem können die viralen Bestandteile selbst immunogen wirken, was zu einer Herabsetzung ihrer Wirksamkeit durch das Immunsystem des Patienten führt. Diese auszugshaften Risiken stehen einer breiten Anwendung als gentherapeutischer Vektor in erheblichem Maße gegenüber.

Mehrere Publikationen zeigen, dass die besten therapeutischen Resultate durch eine Kombination von Zytokingenen mit dem Wachstumsfaktor GM-CSF erzielt werden (Paillard, 1998, Hum. Gene Ther. 9: 2457-2458; Schadendorf et al., 1995, J. Mol. Med. 73: 473-477). Die Bedeutung von GM-CSF bei Tumorantigenimpfungen zeigte sich auch in der Steigerung der klinischen und immunologischen Antitumorantwort bei Peptidvakzinierungen (Jäger et al., 1996). Offenbar spielt dabei die Aktivierung von antigen-präsentierenden Dendritischen Zellen sowie die Stimulation einer Effektorzellpopulation die wesentliche Rolle.

Bislang ist es jedoch unklar, welche Zytokingene in Kombination in einer immunogenen Zusammensetzung mit GM-CSF die effektivste anti-tumorale Immunantwort auslösen.

In Experimenten in Mäusen zeigte sich, daß eine Impfung mit Expressionskonstrukten kodierend für IL-7 einen anti-tumoralen Effekt hervorruft (Miller et al., 1993, Blood 18: 3686-3694; Murphy et al., 1993, J. Clin. Invest. 92: 1918-1924). Ebenso ist bekannt, das die Inkubation von CTLs mit IL-7 bzw. IL-7-transfizierten Zellen zur Tumorregression in Mäusen (Jicha et al., 1991; Hock et al., 1993) führt, und die Transfektion mit IL-7 und B7.1/CD80 CD28⁺CD25⁺ T-Zell-Infiltrate und Immunität induziert (Cayeux et al., 1995).

Bislang gibt es keine therapeutischen Erfolge, nicht einmal im Mausmodell.

Darüberhinaus ist bei Versuchen zur Stimulation von Immunantworten durch Gentherapie mit Plasmid-DNA oder Oligonukleotid-Sequenzen beobachtet worden, daß gewisse Nukleinsäuresequenzen, die CpG-Motive (CpG = unmethyliertes Cytosin-Guanosin-Dinukleotid) aufweisen, eine enorme immunstimulatorische Wirkung haben können (Schmidt-Wolf et al., 1989, J. Immunol. Methods 125: 185-189). Immunstimulatorische Nukleinsäuresequenzen (ISS) sind aus diesem Grunde schon früh als Adjuvantien in DNA-basierten Immunisierungsprotokollen gegen infektiöse Erreger eingesetzt worden (Sato et al., 1996, Science 273: 352-354). In Mausexperimenten zeigte sich, daß die Verabreichung von CpG-reichen DNA-Sequenzen zu einer starken Aktivierung der B-Zellen führt und die Expression bestimmter Zytokine, beispielsweise von IL-6 und GM-CSF, anregt. Ebenso konnte im Mausmodell gezeigt werden, das eine Immunisierung mit CpG-Oligodesoxyribonukleotiden (ODN) gemeinsam mit einem Fusionsprotein drei Tage vor der Tumor-Inokulation ein Tumorwachstum in der Maus verhindern konnte (Liu et al., 1998). Die Lehre von Gebrauch und Herstellung immunstimulatorischer, CpGenthaltender ISS ist in der WO 98/18810 umfassend erläutert.

Aus der WO 00/04918 ist bekannt, Tumorzellen mit Genen, kodierend beispielsweise für Interferon-gamma und GM-CSF, zu transfizieren. Als Expressionsvektoren werden Plasmide eingesetzt. Diese Schrift zeigt ein Konzept auf, TumorErkrankungen per Immuntherapie zu behandeln. Aussagekräftige in-vivo bzw. in-vitro Daten oder klinische Ergebnisse, die die Wirksamkeit der beanspruchten Vakzine belegen, werden nicht gezeigt. Bemerkt sei, dass diese plasmid-basierten Vektoren für eine Anwendung in der humanen Gentherapie nicht ohne Vorbehalt geeignet sind, da sie neben den therapeutischen Sequenzen genetische Funktionseinheiten tragen, die sie für ihre Replikation benötigen. Daneben weisen sie Antibiotika-Resistenzgene auf, die für ihre Selektion unerläßlich sind. Es erfolgt also eine dauernde Expression therapeutisch nicht erwünschter Säuger - oder Bakterienproteine.

Trotz langjährigen Forschungen und erfolgversprechenden Ansätzen, ist es bisher nicht gelungen, eine wirksame immun-basierte Therapie gegen Tumorerkrankungen durch die Applikation von immunogenen Substanzen zu entwickeln.

Aufgabe der Erfindung ist es, eine Vakzine als Arzneimittel zur Behandlung von mit Zytokinen in Zusammenhang stehenden Erkrankungen, wie beispielsweise Krebserkrankungen zur Verfügung zu stellen, die spezifisch und effizient einsetzbar ist und insbesondere zur Induktion von tumorspezifischen Immunantworten führt. Ferner soll ein entsprechendes Verfahren zur Herstellung einer derartigen Vakzine bereitgestellt werden.

Gelöst wird diese Aufgabe durch die Merkmale der selbstständigen Ansprüche.

Im Sinne der Erfindung bedeuten:
- allogen: von einem genetisch differenten Individuum derselben Art (Spezies) im Gegensatz zu "autolog" (körpereigene Zellen)
- APC: Antigen Presenting Cells
- B7.1/CD80: Cluster of Differentiation 80
- CD40L/CD154: Cluster of Differentiation 40 Ligand
- DC: Dendritische Zellen
- DSLIM: double Stem-Loop Immunomodulating Oligodeoxyribonucleotides
- GM-CSF: granulocyte makrophage-colony-stimulating factor
- IL-7: Interleukin-7
- ISS: immunstimulatorische Nukleinsäuresequenzen
- MIDGE: Minimalistic Immunologically Defined Gene Expression Vector (MIDGE^{®} ist eine eingetragene Marke der Mologen AG)
- ODN: Oligodesoxyribonukleotid
- TAA: tumor-assoziierte Antigene

Nachfolgend sollen eine Reihe allgemeiner Begriffe wie folgt verstanden werden:

Transfizierte Zellen im Sinne der Erfindung sind humane allogene Tumorzellen, die ex-vivo mit entsprechend der Erfindung kodierenden Expressionsvektoren behandelt wurden und als Folge dieser Behandlung die kodierten Zytokin- und kostimulatorischen Faktorsequenzen exprimieren und als Immuntherapeutikum bei Tumorerkrankungen eingesetzt werden.

Im Zusammenhang mit der vorliegenden Erfindung betrifft der Begriff kostimulatorischer Faktor und/oder Zytokin sowohl natürlich vorkommende kostimulatorische Faktoren und/oder Zytokine als auch alle Modifikationen, Mutanten oder Derivate der kostimulatorischen Faktoren und/oder Zytokine, mittels Rekombinationstechniken hergestellte kostimulatorische Faktoren und/oder Zytokine, die Aminosäure-Modifikationen, wie Inversionen, Deletionen, Insertionen, Anlagerungen usw. enthalten, sofern zumindest ein Teil der essentiellen Funktionen der Wildtypkostimulatorischen Faktoren und/oder Zytokine vorhanden ist. Solche kostimulatorischen Faktoren und/oder Zytokine können auch ungewöhnliche Aminosäuren und/oder Modifikationen, wie eine Alkylierung, Oxidation, Thiol-Modifikation, Denaturierung und Oligomerisation und dergleichen umfassen. Im Zusammenhang mit der vorliegenden Erfindung können kostimulatorische Faktoren und/oder Zytokine insbesondere Proteine, Peptide und/oder Fusionspeptide sein, die neben anderen Proteinen, Peptiden oder Teilen davon kostimulatorischen Faktoren und/oder Zytokine insgesamt oder teilweise enthalten. In einer weiteren Ausführungsform der vorliegenden Erfindung sind die kostimulatorischen Faktoren und/oder Zytokine verkürzte Formen der natürlich vorkommenden kostimulatorischen Faktoren und/oder Zytokine.

Alle oben genannten kostimulatorischen Faktoren, die geeignet sind, die Reaktion des Immunsystems zu modulieren, sind im Sinne dieser Erfindung immunogene Substanzen. Eine erfindungsgemäße Vakzine stellt im Sinne dieser Erfindung folglich eine immunogene Zusammensetzungen dar, da sie eine Kombination von immunogenen Substanzen enthält. Tumorzellen unterscheiden sich von normalen Zellen unter anderem in einer veränderten Expression von Zelloberflächenproteinen. Vor allem die Expression von tumor-assoziierten Antigenen (TAA) erlaubt theoretisch die Erkennung und Vernichtung dieser Zellen durch das Immunsystem. Häufig ist jedoch das Immunsystem der Patienten supprimiert, so dass es ihm nicht gelingt, die veränderten Zellen zu erkennen.

Dieses Problem wird durch die Bereitstellung einer Vakzine zur Behandlung von Patienten mit definierten Tumorerkrankungen gelöst, wobei diese Vakzine aus Tumorzellen eines anderen Patienten (allogene Zellen) besteht und diese Tumorzellen zuvor ex-vivo mit für Interleukin-7 (IL-7), für Granulozyten-Makrophagen-Koloniestimulierenden Faktor GM-CSF (granulocyte makrophage-colony-stimulating factor), und für die kostimulatorischen Faktoren CD40UCD154 und B7.1/CD80 kodierenden Expressionskonstrukten transfiziert wurden. In einer weiteren Ausführungsform der Erfindung wird eine entsprechende Vakzine geschaffen, wobei diese Tumorzellen zuvor ex-vivo mit für Interleukin-7 (IL-7), für Granulozyten-Makrophagen-Koloniestimulierenden Faktor GM-CSF (granulocyte makrophage-colony-stimulating factor) und für den kostimulatorischen Faktor CD40UCD154 kodierenden Expressionskonstrukten transfiziert wurde.

Bei den verwendeten Tumorzellen kann es sich um solche handeln, die von Patienten mit gleichem Krankheitsbild wie der zu behandelnde Patient stammen, oder aber um solche, die von Patienten mit anderem Krankheitsbild wie der zu behandelnde Patient stammen.

Die Kombination der Bestandteile (immunogene Substanzen) der Vakzine soll gewährleisteten, dass alle drei Stufen der Signalkaskade, die grundsätzlich zur Induktion einer spezifischen Immunantwort notwendig sind, erzeugt werden. Die drei Stufen umfassen die Antigenpräsentation, die Kostimulation und die Bereitstellung einer geeigneten lokalen Umgebung.

Die Expression von GM-CSF und CD40UCD154 soll bewirken, dass antigenpräsentierende Zellen (APC) sowie Dendritische Zellen (DC) lokal rekrutiert und aktiviert werden. Als Folge kommt es zu einer verstärkten Präsentation von TAA.

Die Expression von B7.1/CD80 führt zu einer Steigerung der kostimulatorischen Aktivität. Dadurch wird die Antigenität der TAA verstärkt und die Anzahl der gegen TAA aktivierten T-Lymphozyten gesteigert.

Die Expression von IL-7 induziert zusätzlich die Proliferation der tumor-spezifischen T-Lymphozyten.

Die erfindungsgemäße Vakzine führt somit am Applikationsort zu einer hohen Konzentration an löslichen proliferationsfördernden Zytokinen und kostimulatorischen Molekülen im Verbund mit den TAA auf der Oberfläche der Tumorzellen, die zu Aktivierung und Proliferation von tumor-spezifischen T-Zellen führt. Ebenso werden APC und DC an den Applikationsort "gelockt".

Die Expressionskonstrukte liegen entweder als Plasmid vor, bevorzugt sind aber auch minimalistische immunologisch definierte Genexpressionskonstrukte. Dabei handelt es sich um eine linear-doppelsträngige, kovalent geschlossene Expressionskassette, die lediglich aus einem CMV-Promotor, einem Intron, der kodierenden Gensequenz und einer Polyadenylierungssequenz besteht. Die Expressionskassette ist an beiden Enden des Doppelstrangs durch eine kurze Schleife einzelsträngiger Nukleotidreste kovalent geschlossen. Diese kovalent geschlossenen minimalistische DNA-Konstrukte werden im Folgenden als MIDGE-Vektoren bezeichnet (MIDGE: Minimalistic Immunologically Defined Gene Expression Vector); vgl. EP 0 941 318 B1. Die MIDGE-Konstrukte haben den Vorteil, dass mit ihnen auf Strukturen verzichtet werden kann, die für die therapeutische Wirkung nicht essentiell sind, was letztlich die Nachteile herkömmlicher Genfähren vermeidet.

Die Bereitstellung einer lokalen Umgebung, die permissiv für die Induktion einer spezifischen Immunantwort ist, wird durch den Einsatz eines Adjuvanz in Form von immunstimulatorischen Nukleinsäuresequenzen (ISS) gewährleistet. Zu diesem Zweck ist es erfindungsgemäß vorgesehen, dass die Vakzine zusätzlich immunmodulierende Oligodesoxyribonukleotide als Adjuvanz umfasst. Bevorzugt ist hierbei ein immunmodulierendes Oligodesoxyribonukleotid, welches
a) eine Sequenz mit der Basenfolge N¹N²CGN³N⁴ umfasst, wobei N¹N² ein Element ausgewählt aus der Gruppe umfassend GT, GG, GA, AT oder AA, N³N⁴ ein Element ausgewählt aus der Gruppe umfassend CT oder TT, sowie C Desoxycytosin, G Desoxyguanosin, A Desoxyadenosin und T Desoxythymidin ist,
b) und einen zirkulären Strang Desoxyribonukleinsäure mit einer teilweise zueinander komplementären, antiparallelen Basensequenz umfasst und hantelförmig ausgebildet ist.

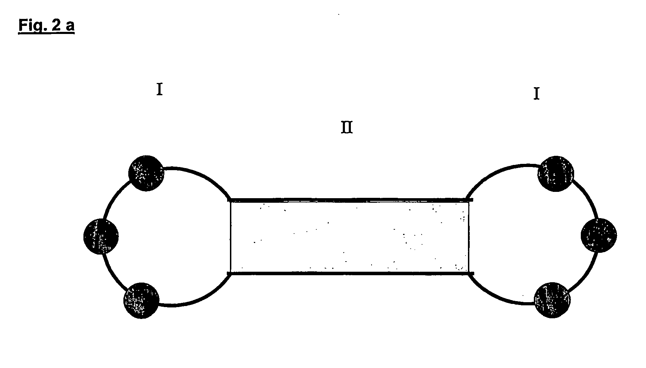

Die CpG-Motive der ISS (siehe hierzu Fig. 2a) bewirken eine Erhöhung der Aktivität von NK-Zellen und Makrophagen sowie eine starke Stimulation der zellulären TH1-Immunantwort. Sie wirken somit als Immunmodulatoren und erlauben und verstärken die tumor-spezifische Immunantwort. Bevorzugt werden kovalent geschlossene ISS mit einer Länge von 30 bp eingesetzt, wie sie in der WO 01/07055 beschrieben werden. Die Konstrukte werden im Weiteren als dSLIM (double Stem-Loop Immunomodulating Oligodeoxyribonucleotides) bezeichnet.

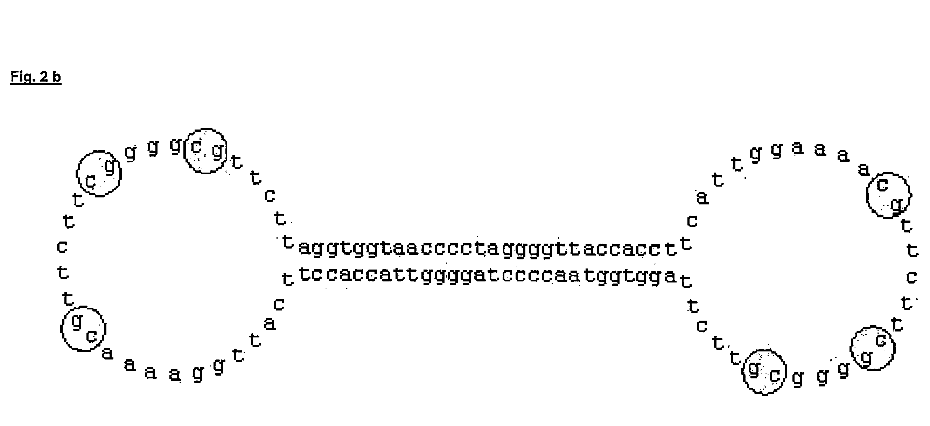

Die Sequenz mit der Basenfolge N¹N²CGN³N⁴ ist im einsträngigen Bereich des Oligodesoxyribonukleotids positioniert und umfasst 40 bis 200 Nukleotide (siehe Fig. 2b).

Gegenstand der Erfindung ist auch ein Verfahren zur Herstellung einer oben beschriebenen Vakzine zur Behandlung von Patienten mit Tumorerkrankungen, enthaltend allogene Tumorzellen und immunmodulierende Oligodesoxyribonukleotide, wobei die Tumorzellen zuvor ex-vivo mit
a) für Interleukin-7 (IL-7), Granulozyten-Makrophagen-Koloniestimulierenden Faktor (GM-CSF), CD40UCD154 und B7.1/CD80, oder
b) für Interleukin-7 (IL-7), Granulozyten-Makrophagen-Koloniestimulierenden Faktor (GM-CSF) und CD40L/CD154
kodierenden Nukleinsäuremolekülen transfiziert werden und anschließend in eine applikationsfähige pharmazeutische Zusammensetzung überführt werden. Die bereits oben beschriebenen immunmodulierenden Oligodesoxyribonukleotide umfassen einen zirkulären Strang Desoxyribonukleinsäure mit einer teilweise zueinander komplementären, antiparallelen Basensequenz und sind hantelförmig ausgebildet. Sie sind als Adjuvanz ebenfalls Bestandteil des Verfahrens.

Erfindungsgemäß ist mithin vorgesehen, dass eine allogene Tumorzelle mindestens drei, vorzugsweise vier Nukleinsäuremoleküle, die für die kostimulatorischen Faktoren B7.1/CD80 und CD40UCD154 und die Zytokine Interleukin-7 und GM-CSF gemeinsam kodieren, umfasst.

Gegenstand der Erfindung sind daher auch dementsprechend transfizierte, allogene humane Tumorzellen, die ex-vivo mit
a) für Interleukin-7 (IL-7), Granulozyten-Makrophagen-Koloniestimulierenden Faktor (GM-CSF), CD40UCD154 und B7.1/CD80, oder
b) für Interleukin-7 (IL-7), Granulozyten-Makrophagen-Koloniestimulierendern Faktor (GM-CSF) und CD40L/CD154
kodierenden Nukleinsäuremolekülen transfiziert wurden und entsprechende Expressionskonstrukte aufweisen. Die Tumorzellen weisen Expressionskonstrukte in Form eines Plasmids oder aber als linear-doppelsträngige, kovalent geschlossene Expressionskassette auf, die lediglich aus einem CMV-Promotor, einem Intron, der kodierenden Gensequenz und einer Polyadenylierungssequenz besteht, welche an beiden Enden des Doppelstrangs durch eine kurze Schleife einzelsträngiger Nukleotidreste kovalent geschlossen ist. Es handelt sich vorzugsweise um eine allogene Tumorzelle einer Nierenzellkarzinomzelllinie.

Wie bei der Vakzine selbst ist auch beim erfindungsgemäßen Verfahren vorgesehen, dass die Nukleinsäuremoleküle in einem oder mehreren Expressionskonstrukten vorliegen. Es kann sich mithin um ein DNA-Expressionskonstrukt zur multiplen Genexpression handeln,
- bestehend aus doppelsträngigen Bereichen, die eine lineare Expressionskassette enthalten, welche zumindest eine Promotorsequenz und eine kodierende Sequenz aufweisen,
- und wobei diese doppelsträngigen Bereiche durch DNA-Einzelstränge oder nicht-kodierende DNA-Doppelstränge miteinander verbunden sind,
- und die Expressionskassetten an den Enden, an denen sie nicht mit weiteren Expressionskassetten über DNA-Einzelstränge oder nicht-kodierende DNA-Doppelstränge miteinander verbunden sind, gegen Exonukleaseabbau geschützt sind.

Ein solches DNA-Expressionskonstrukt zur multiplen Genexpression ist bereits in der PCT/DE03/02478 beschrieben. Mithin können mehrere Nukleinsäuren, die Gegenstand der erfindungsgemäßen Vakzine sind, in einem einzigen Expressionskonstrukt angeordnet sein.

In einer besonders bevorzugten Ausführungsform der Erfindung ist das Nukleinsäuremolekül eine DNA, insbesondere eine cDNA oder eine genomische DNA. Selbstverständlich kann es auch vorteilhaft sein, dass das Nukleinsäuremolekül eine RNA ist.

Die allogenen Tumorzellen stammen von Patienten mit kolorektalem Karzinom, kleinzelligem und nicht-kleinzelligem Lungenkarzinom, Prostatakarzinom, Mammakarzinom, Ovarialkarzinom und Nierenzellkarzinom, sowie malignem Melanom.

Im Rahmen der Erfindung wurde insbesondere eine Nierenzellkarzinomzelllinie verwendet, die sich besonders zur Herstellung der erfindungsgemäßen Vakzine eignet. Eine bevorzugte Nierenzellkarzinomzelllinie wurde bei der DSMZ (Deutsche Sammlung von Mikroorganismen und Zellkulturen GmbH) als lebensfähige Kultur unter der Nummer DSM ACC 2635 hinterlegt. In Zellen dieser Nierenzellkarzinomzelllinie werden mittels im Wesentlichen bekannter Transfektionsmethoden biologischer, chemischer und/oder physikalischer Art die DNA-Expressionskonstrukte eingebracht, die in den Zellen zur Expression der erwünschten Gene führen. Zu den derart transfizierten allogenen Zellen wird ein Adjuvanz, bevorzugt ISS, besonders bevorzugt dSLIM (siehe oben), gegeben. Nach radioaktiver Bestrahlung der transfizierten allogenen Tumorzellen mit Gamma-Strahlung wird die erfindungsgemäße Vakzine Patienten mit einer Tumorerkrankung appliziert.

Der Körper der Zellentnahme ist - im Unterschied zur Therapie mit autologen Zellen - nicht der mit dem Arzneimittel selbst zu behandelnde Körper. Die Verwendung von allogenen Zellen hat einerseits den Vorteil der einmaligen Charakterisierung und verlässlichen Vermehrung der Zellen, andererseits wird der allogene Stimulus als zusätzliche günstige Adjuvanzwirkung angesehen.

Die zu behandelnden Zellen können aus einem einzigen (fremden) Körper stammen oder von mehreren Körpern mit gleichem Krankheitsbild vereint (gepoolt) werden.

Die Erfindung betrifft somit die Kombination von drei oder vier exprimierbaren Nukleinsäuren, die für zwei Zytokine und ein oder zwei kostimulatorische Faktoren kodieren.

Als zusätzlicher Immunmodulator können ISS, bevorzugt dSLIM, eingesetzt werden.

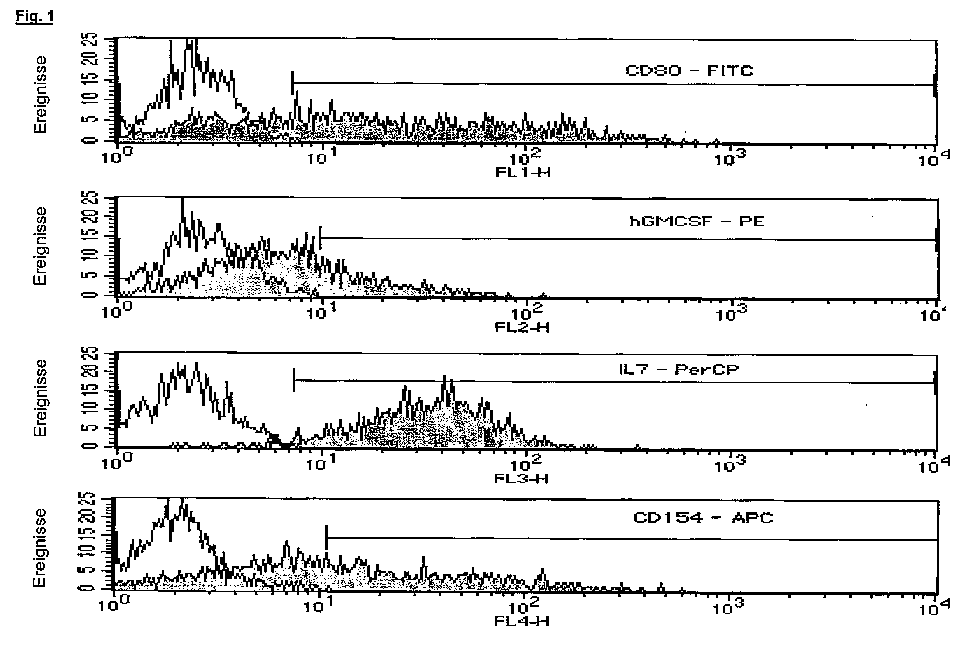

In Zellkulturexperimenten in primären humanen Zelllinien konnten nach der Transfektion dreifach bzw. vierfach positive Zellen nachgewiesen werden (siehe Fig. 1). "Dreifach" bzw. "vierfach" positiv heißt in diesem Zusammenhang, dass ein hoher Prozentsatz der Zellen mit allen drei oder vier Genen, kodierend für GM-CSF, IL-7, CD40UCD154 und B7.1/CD80 zugleich erfolgreich transfiziert wurde und diese drei oder vier Gene dann von diesen Zellen exprimiert wurden. Bei der verwendeten Kolonkarzinomzelllinie konnten 14,4% aller Zellen als vierfach positive Zellen mittels FACS-Analyse (Flourescence Aktivated Cell Sorter) bestimmt werden. Ferner konnte festgestellt werden, dass unabhängig vom Gen 20,3% aller Zellen nach der Elektroporation mit einem Gen transfiziert waren; mit drei verschiedenen Genen waren noch 19,9% der Zellen erfolgreich transfiziert. Es wurde festgestellt, dass der prozentuale Anteil der erfolgreich transfizierten Kolonkarzinomzellen nach der Vierfach-Transfektion mit Expressionskonstrukten kodierend für CD40L/CD154, GM-CSF, B7.1/CD80 und IL-7 bei 14,4 % der Zellen liegt. 14,4% der Zellen waren demnach mit vier verschiedenen Genen transfiziert und in der Lage, diese erfolgreich zu exprimieren. Insgesamt waren 69,4% aller Zellen mit einem oder mehreren der Gene transfiziert.

Die Transfektion mit mehreren Genen ist von Vorteil, da so die exprimierten Zytokine und kostimulatorischen Faktoren in enger räumlicher Nähe zueinander in den Effektororganen des Immunsystems exprimiert werden. Diese räumliche Nähe ist bedeutsam, da kostimulatorische Signale nur in Zusammenhang mit Antigenen wirksame Verstärkung der impfung liefern.

Zur Transfektion können im Wesentlichen bekannte biologische, chemische und/oder physikalische Methoden eingesetzt werden, beispielsweise Transfektion mittels ballistischem Transfer (EP 0 686 697), Polykationen-Transfektion, Kalziumphosphatpräzipitation, Mikroinjektion, Protoplastenfusion, Liposomenfusion, virale Transfektionssysteme, Lipofektion und/oder Elektroporation in-vivo und/oder in-vitro. In einer bevorzugten Ausführungsform der Erfindung erfolgt die Transfektion mittels Elektroporation.

Weitere vorteilhafte Methoden sind die biologischen Transfektionsmethoden wie der rezeptorvermittelte Gentransfer. Hierbei beispielsweise werden die DNA-Expressionkonstrukte, die für mindestens einen, vorzugsweise zwei kostimulatorische Faktoren und zwei Zytokine kodieren, kovalent mit einem Oligopeptid verbunden, welches vorzugsweise die Kern-Lokalisierungssequenz (Nuclear Localization Signal = NLS) aus dem Simian Virus SV-40 ist.

Durch die Verwendung anderer, zum Teil synthetisch hergestellter Peptide ist es möglich, die Transfektionseffizienz weiter zu steigern. Diese Methode eignet sich insbesondere für den zellspezifischen in-vivo-Gentransfer nach intravenöser Applikation.

Die durch das erfindungsgemäße Verfahren hergestellte erfindungsgemäße Vakzine aus modifizierten allogenen Tumorzellen zeigt überraschende Wirkungen. Vorteilhafterweise können sowohl Defizite im Immunsystem von Tumorpatienten beseitigt und gleichzeitig die intrinsisch vorhandene Immunantwort auf die tumor-spezifischen Antigene verstärkt werden.

Weiterhin kann die Vakzine bei Patienten, deren Tumor entfernt wurde, als adjuvante Therapie zur Verbesserung und Wiederherstellung ihrer Immunantwort gegen residuale Tumorzellen oder zur Verringerung der verbliebenen Tumormasse eingesetzt werden. Eine Perspektive öffnet sich für Patienten mit chirurgisch behandeltem Primärtumor ohne offensichtliche Metastasen, die von einer additiven tumor-spezifischen Therapie, welche die Rezidivrate vermindert, profitieren könnten.

Weitere vorteilhafte Ausgestaltungsformen der Erfindung ergeben sich aus den Unteransprüchen und der Beschreibung. Die Erfindung einschließlich der überraschenden Wirkung des erfindungsgemäßen Arzneimittels (als Impfstoff zur Karzinomtherapie), sowie des erfindungsmäße Verfahrens wird anhand der Abbildungen und den Ausführungsbeispielen nachfolgend näher beschrieben; es zeigt:
- Fig. 1: FACS-Intensitäten von vierfach transfizierten humanen Kolonkarzinomzellen. Die Transfektion erfolgte durch Elektroporation mit Expressionsvektoren kodierend für CD40UCD154, GM-CSF, B7.1/CD80 und IL-7;
- Fig. 2a und b: Schema der immunmodulatoren (dSLIM): hantelförmige Struktur mit jeweils drei, schematisch dargestellten CG-Motiven in den Haarna- delstrukturen der Loops; darin bedeuten I: Loop und II: Stamm.

Für den Nachweis der Genprodukte wurden Antikörper verwendet, an die vier unterscheidbare Fluoreszenz-Farbstoffe gekoppelt sind. In Fig.1 mit
CD80-FITC
hGMCSF-PE
IL7-PerCP
CD154-APC bezeichnet.

Der Fluorescence Activated Cell Sorter (FACS) erkennt diese Farben in vier verschiedenen Kanälen. Auf der Abzisse mit FL1-H, FL2-H, FL3-H und FL4-H bezeichnet. Die Exponenten auf der Abzisse geben eine relative Fluoreszenz-Intensität an. Auf der Ordinate sind die Counts angegeben, die entsprechen ungefähr der Anzahl der gemessenen Zellen. Die Auswertung erfolgt durch das sogenannte Gating, das heißt, es werden nur die Zellen gezählt, die durch ein bestimmtes Energie-Tor passen.

In der folgenden Tabelle 1 ist der prozentuale Anteil der erfolgreich transfizierten allogenen Nierenzellkarzinomzellen nach der Dreifach-Transfektion mit Expressionskonstrukten kodierend für CD40L/CD154, GM-CSF und IL-7 bzw. Vierfach-Transfektion mit Expressionskonstrukten kodierend für CD40UCD154, GM-CSF, B7.1/CD80 und IL-7 dargestellt.

**Tab. 1:**

| Kodierendes Gen | Dreifach-Transfektion mit Expressionskonstrukten kodierend für CD40L/CD154_{,} GM-CSF und IL-7 | Vierfach-Transfektion mit Expressionskonstrukten kodierend für CD40L/CD154, GM-CSF, B7.1/CD80 und IL-7 |
|---|---|---|
| | Anteil transfizierter Zellen (1 Messung) | Anteil transfizierter Zellen (2 Messungen) |
| B7.1/CD80 | --- | 66,3 bis 87,6 % |
| CD40UCD154 | 60,8 % | 21,6 bis 29,5 % |
| IL-7 | 21,7 ng in 1 x 10⁶ Zellen | 14,9 bis 38,2 ng in 1 x 10⁸ Zellen |
| GM-CSF | 698,9 ng in 1 x 10⁶ Zellen | 370,6 bis 1.567,2 ng in 1 x 10⁶ Zellen |

Die Transfektion der ko-stimulatorischen Faktoren B7.1/CD80 bzw. CD40UCD154 wurde durch Färbung der Zellen mit fluoreszenzmarkierten Antikörpern nachgewiesen; das Ausmaß der Transfektion mit Zytokinen IL-7 bzw. GM-CSF wurde anhand der Konzentration der Zytokine im Medium der Zellkultur nachgewiesen.

Die erfindungsgemäße Vakzine, bestehend aus allogenen, ex-vivo transfizierten Zellen wurde Patienten mit metastasierten soliden Tumoren, im Abstand von 7 bis 10 Tagen in Fom von 4 Injektionszyklen bestehend aus jeweils 2 Injektionen verabreicht. Die Zellen waren zuvor mit Expressionsvektoren kodierend für IL-7, GM-CSF, CD40UCD154 und/oder B7.1/CD80 transfiziert und im Anschluß mit immunstimulatorischen Sequenzen (ISS) versetzt wurden. Die zwei ersten Injektionen erfolgten am Tag 1 in die typischen Impfstellen in der Haut (intradermal) seitlich am linken und rechten Oberarm, am Tag 7 (bis 10) in die typischen Impfstellen intradermal auf der Vorderseite des linken und rechten Oberschenkels, am Tag 14 (bis 20) unter die Haut (subkutan) ca. 30mm links und rechts des Bauchnabels und am Tag 21 (bis 30) wieder in die Oberarme aber unter die Haut (subkutan).

Alle Patienten befanden sich zu Beginn der Behandlung in einer fortgeschrittenen Phase der Erkrankung. Zur Beurteilung des klinischen Erfolges sollen folgende Formulierungen entsprechend World Health Organization (1979) gelten: als eine partielle Reaktion (partial Response, PR) wird der Rückgang der erfaßbaren Tumorherde um mehr als 50% in den letzten vier Wochen sowie die Unterdrückung neuer Tumorherde bezeichnet. Unter stabilem Krankheitsverlauf (stable disease, SD) ist das Gleichbleiben des Zustandes zu verstehen. Eine mixed Response, (MR), ist eine gemischte Reaktion, bspw. das Fortschreiten der Metastierung und ein Rückgang der Metastasen an einem anderen Ort. Eine complete Response (CR) bezeichnet den vollständigen Rückgang der Tumorherde.

Im Folgenden soll die Erfindung anhand von Beispielen näher erläutert werden, ohne dass aber die Erfindung auf diese Beispiele zu beschränken ist.

### Vakzinierung

Der Patient, männlich, an kleinzelligem Lungenkarzinom erkrankt, erhielt im Abstand von 7 Tagen vier aufeinanderfolgende Impfungen mit der erfindungsgemäßen Vakzine. Die allogene Nierenzellkarzinomzelllinie wurde wie zuvor beschrieben behandelt. Eine Expressionskontrolle erfolgte 24h nach der Transfektion. Die Kontrolle der Expression der kostimulatorischen Faktoren B7.1/CD80 bzw. CD40UCD154 erfolgte durch Färbung der Zellen mit fluoreszenzmarkierten Antikörpern und nachfolgender durchflußzytometrischer Messung. Die Bestimmung der Expression der Zytokine IL-7 und GM-CSF erfolgte durch Konzentrationsmessung im Medium der Zellkultur mit Hilfe von ELISA.

Erfolgreich transfiziert bedeutet: lebende positive Zellen. Die Daten der Transfektion sind in der Tabelle 1 dargestellt. Der prozentuale Anteil an mit B7.1/CD80 erfolgreich transfizierten Zellen betrug zwischen 66,3 und 87,6 %. Der Anteil an mit CD40UCD154 transfizierten Zellen zwischen 21,6 und 29,5 %. Die Konzentration des Zytokins IL-7 betrug in 1x10⁶ Zellen zwischen 14,9 und 38,2 ng. Die Konzentration an GM-CSF betrug in 1x10⁶Zellen zwischen 370,6 und 1.567,2 ng.

Im Fall der Transfektion zur Dreifach-Vakzinierung mit Expressionskonstrukten kodierend für CD40UCD154, GM-CSF und IL-7 lag der prozentuale Anteil an mit CD40L/CD154 erfolgreich transfizierten Zellen bei 60,8 %. Die Konzentration des Zytokins IL-7 betrug in 1x10⁸ Zellen 21,7 ng. Die Konzentration an GM-CSF betrug in 1x10⁶ Zellen 698,9 ng.

### Behandlung von metastasierenden Tumorerkrankungen

Auswahl der Patienten: Patienten mit kolorektalem Karzinom, kleinzelligem und nicht-kleinzelligem Lungenkarzinom, Prostatakarzinom, Mammakarzinom, Ovarialkarzinom und Nierenzellkarzinom, sowie malignem Melanom., im Alter zwischen 18 und 70 Jahren, und einem Karnofsky-Index von 70-100 waren aufnahmeberechtigt. Ausschlusskriterium waren: eine vorhergegangene Zytokinbehandlung oder Chemotherapie, die weniger als 28 Tage zurücklag, Kreatinwerte über 265Mikromol/l, Bilirubinwerte über 51 Mikromol/l, nichtkompensierte Herzinsuffizienz, ventrikuläre Rhytmusstörungen, schwere psychatrische Krankheiten, aktive Hepatitis A, B oder C, HIV Infektion.

Behandlung: Neben der Anamnese wurden die folgenden Parameter vor Begin der Behandlung bestimmt: physische Untersuchung, Hämatologie (Hämoglobin, Hematocrit, Leukozyten und Plättchenzahl), chemische Blutwerte und Urinanalyse. Es wurde für die Immunstatusbestimmung Blut abgenommen. DHT (Delayed type hypersensitivity) Hauttests wurden mit dem Multitest Merieux Test (Leimen, Germany) durchgeführt. Röntgenaufnahmen des Oberkörpers sowie Computertomographien des Oberkörpers und Abdomens wurden erstellt. Die Patienten erhielten vier subkutane Injektionen mit mindestens 8x10⁸-1,4x10⁷ Zellen mit zuvor ex-vivo behandelten Tumorzellen. Umfassende immunologische Untersuchungen, hämatologische Untersuchungen und grobe klinische Untersuchungen (physische Untersuchung, Ultraschall Untersuchung und Untersuchung des Unterleibes, bei Bedarf) wurden am 14., 28. und 56. Tag wiederholt. Eine komplette klinische und immunologische Untersuchung, vergleichbar mit der eingangs durchgeführten Auswahluntersuchung, fand am 84. Tag statt.

### Minimalistische immunologisch definierte Genexpressionskonstrukte (MIDGE)

Kovalent geschlossene minimalistische DNA-Expressionsvektoren wurden zur Transfektion der Tumorzellen eingesetzt. Die Expressionsvektoren dienten der Expression des menschlichen interleukin-7 (IL-7), des menschlichen Granulozyten-Makrophagen-Koloniestimulierender Faktors (GM-CSF), der kostimulatorischen Faktoren B7.1/CD80 und CD40UCD154. Die MIDGE-Vektoren wurden nach SOP-Vorschrift und in einem der Klasse B entsprechendem Labor mit anschließender Qualitätskontrolle synthetisiert.

Die kodierenden Sequenzen für IL-7 (NCBI Acc. Nr.:J04156), GM-CSF (NCBI Acc.Nr.:M11220), B7.1/CD80 (NCBI Acc.Nr.:AF177937) und CD40UCD154 (NCBI Acc.Nr.:S49008, entnommen der Datenbank (www.ncbi.nlm.nih.gov) des National Center for Biotechnology Information), wurden in pMOK-Plasmide inseriert und dienten als Ausgangskonstrukte für die Herstellung der MIDGE-Vektoren. Das Plasmid pMOK besitzt neben dem starken "early immediate promotor" aus dem CMV-Virus, ein Intron und eine Polyadenylierungssequenz aus dem SV40-Virus (MOLOGEN, Berlin). Das Plasmid pMOK kodierend für IL-7 wurde mit dem Restriktionsenzym Eco31I über Nacht bei 37°C vollständig verdaut. Durch den Restriktionsverdau wurden zwei DNA-Fragmente erzeugt. Das eine bestand aus dem Kanamycin-Resistenzgen, sowie anderen zur Propagierung des Plasmides in Bakterien notwendigen Sequenzen. Das andere Fragment bestand aus den Sequenzen, die Bestandteil der MIDGE-DNA werden sollten: enhanced CMV-Promotor, chimäres Intron, der Gensequenz für IL-7 und der Polyadenylierungssequenz aus SV-40. Mittels des Enzymes T4-DNA Ligase wurden die 5' phosphorylierten haarnadelförmigen Oligodesoxyribonukleotide (TIB-MolBiol, Berlin, ODN 1 = Seq.ID 5 aus WO 03/031469 A2 (Mologen et al.) ohne modifizierte Base X) und ODN 2 = Seq.ID 6 aus WO 03/031469 A2 (Mologen et al.)) in Anwesenheit des Restriktionsenzymes Eco31I über Nacht bei 37°C an das die MIDGE-DNA bildende DNA Fragment ligiert. Insoweit wird bezug genommen auf die in der WO 03/031469 A2 (Mologen et al.) gezeigte Darstellung und die dortigen Sequenzen der Oligonukleotide (ODN). Die Reaktion wurde durch Erhitzen auf 70°C gestoppt. Das resultierende Gemisch an Nukleinsäuren wurde mit dem Enzym T7-DNA-Polymerase behandelt. Die MIDGE-DNA wurde durch Anionenaustauschchromatographie aufgereinigt und mit Isopropanol gefällt (vgl. EP 0 941 318 B1).

Die Herstellung der Vektoren kodierend für GM-CSF, B7.1/CD80 und CD40L/CD154 erfolgte entsprechend.

### Herstellung von NLS-gekoppelten MIDGE

Für die Herstellung von NLS-MIDGE wird bezug genommen auf die in der WO 03/031469 A2 (Mologen et al.) gezeigte Darstellung und die dortigen Sequenzen der Oligonukleotide (ODN). Das NLS-Peptid (Seq.ID 4 aus WO 03/031469 A2 (Mologen et al.)) wird zuerst an die ODN gekoppelt. Dafür wurde das in der Haarnadelschlaufe mit einem aminogruppenmodifizierten desoxy-Uracil (XT) versehene ODN 1 sowie das nichtmodifizierte ODN 2 aus der WO 03/031469 A2 (Mologen et al.) verwendet:

Zuerst wurde das aminomodifizierte Oligonukleotid ODN 1 (0,1 mM) mit sulfo-KMUS (5mM) in PBS bei Raumtemperatur aktiviert. Nach 120 min. wurde die Reaktion mit 50 mM Tris-(Hydroxymethyl)-Aminomethane gestoppt und das aktivierte ODN 1 nach Ethanolpräzipation (300mM NaOAc pH 5.2, 5.5 mM MgCl₂, 70 % Ethanol), Zentrifugation und einmaligem Waschen mit 70% Ethanol erhalten. Das so erhaltene ODN 1 (0, 1 mM) wurde in PBS gelöst und reagierte mit dem NLS-Peptid (0,2mM) für eine Stunde bei Raumtemperatur. Die Reaktion wurde durch Gelektrophorese (3%) und Ethidiumbromidfärbung überprüft. Das entstandene NLS gekoppelte ODN 1 wurde durch HPLC aufgereinigt und mit dem ODN 2 zur Synthese der MIDGE-NLS Konstrukte wie zuvor beschrieben verwendet.

### Doppelsträngige immunolatorische Oligodeoxynukleotide (d-SLIM)

Doppelsträngige immunolatorische Oligodeoxynukleotide sind Moleküle mit CG-Sequenzen. Hierzu werden lineare Oligodesoxynukleotide (ODN) mittels Nukleotid-Loop kovalent geschlossen, so dass sie vor Degradation durch Exonukleasen geschützt sind. Dabei ergeben sich hantelförmige Moleküle, die dSLIM, "double stem-loop immunomodulator" heißen (siehe Fig. 2). Die immunmodulierende Wirkung beruht auf einer unspezifischen Aktivierung des Immunsystems durch die nichtmethylierten CG-Sequenzen, die an Toll-like Rezeptoren binden. Jeder Loop der DSLIM enthält drei nicht-methylierte CG-Motive.

Doppelsträngige Immunomodulatoren d-SLIMs des ISS30 Typs wurden nach (SOP) und abschließender Qualitätskontrolle im Klasse B Labor hergestellt. Hierzu werden einzelsträngige, haarnadelförmige, 5'-phosphorylierte Oligodesoxyribonukleotide (ODN) mit T4-DNA-Ligase ligiert. Nach Verdau der restlichen Edukte mit T7-Polymerase and chromatographischer Aufreinigung, wurden die ODN durch Ethanol und Sodium-Magnesium Acetat Fällung konzentriert und in PBS gelöst. Das genauer Verfahren ist in der WO 01/07055 dargestellt. Fig. 2b zeigt ein solches d-SLIM.

### Charakterisierung der allogenen Tumorzelllinie

Die verwendete Zelllinie stammt aus einem primären Nierenzellkarzinom einer weiblichen Patientin. Aus diesen Primärzellen wurde eine Zelllinie angelegt und wurde bei der DSMZ (Deutsche Sammlung von Mikroorganismen und Zellkulturen GmbH) als lebensfähige Kultur unter der Nummer DSM ACC2635 hinterlegt. Nach einem Screeningverfahren wurden aus angelegten Primärkulturen der Tumorpatientin solche Zellen bestimmt, die als Ausgangsmaterial zur Herstellung der Masterzellbank dienten. Die Zellpopulation musste sich durch die stabile Expression von TAA, durch eine konstante und gute Teilungsrate sowie ein möglichst homogenes Wachstumsverhalten auszeichnen. Ebenso musste die Abwesenheit viraler oder bakterieller Kontamination gewährleistet sein.

Die Nierenzellkarzinomzelllinie wurde von einem zertifizierten Labor negativ auf HI-Viren, Hepatitis B u. C-Viren getestet. Ebenso erfolgte eine Charakterisierung hinsichtlich des HLA-Typs, der Expression von TAA und der Freisetzung von Zytokinen. Zu diesem Zweck wurden verschiedene Techniken wie ELISA's für Zytokinbestimmungen z.B. von: TNF-alpha, IL-6, GM-CSF und IL-7 etabliert. Weiterhin intrazelluläre FACS-Messungen und Echtzeit-RT-PCR-Assays entwickelt. Nach der Transfektion wurden die Zellen auf die Expression von Oberflächenantigenen, Signaltransduktionsmolekülen und Reportergenen untersucht. Die Zelllinie ist durch folgende immunologische Werte spezifischer Oberflächenmarker charakterisiert:

| | |
|---|---|
| EpCAM: | positiv |
| Her-2/neu: | negativ |
| Cytokeratin 7+8: | positiv |
| HLA-A1: | positiv |
| HLA-A2: | positiv |
| Fibroblasten: | negativ |

### Bestimmung der tumorspezifischen Antigene:

| | | | |
|---|---|---|---|
| PRAME: | positiv | SCP-1: | positiv |
| MAGE-4: | negativ | Fertilin-beta: | negativ |
| MAGE-3: | negativ | RAGE-1: | positiv |
| MAGE-1: | negativ | G250: | positiv |

### Elektroporation

Für den ex-vivo Gen Transfer in den Zellkern der Tumorzellen und der Kontrollzellen wurde die Methode der Elektroporation bevorzugt angewendet. Die Tumorzellen werden mit Trypsin/EDTA-Lösung vom Kulturbehälter abgelöst und auf eine Dichte von 1-1.5x10⁷ Zellen pro 600 µl in Medium (ohne FCS) oder PBS eingestellt. Diese 600 µl Zellsuspension wird mit jeweils 5-10 µg Expressionsvektor versetzt, in gekühlte Elektroporationsküvetten (Eurogentec) überführt und bei einer Spannung von 300V und der Kapazität von 1050 Mikrofarad in einem Easyject Elektroporationsgerät (EquiBio) elektroporiert.

Danach werden die Zellen in frisches Medium (5-20ml) überführt und in einem Zellkulturbrutschrank (5% CO₂ 37°C, gesättigte Feuchtigkeit) 24h inkubiert. Es folgte die Messung der Oberflächenmoleküle (CD40UCD154 und B7.1/CD80) und der Zytokine (GM-CSF und IL-7) mittels Vierfarb-Durchflusszytometrie. Ebenso wird eine Zellzahl-u. Vitalitätskontrolle durchgeführt.

Sofort nach dem Gentransfer wurden die Tumorzellen mit 10 ml vorgewärmten Zellsuspensionsmedium bedeckt und durch vorsichtiges Abpipettieren in 15ml Zentrifugenröhrchen überführt. Nach Sedimentation der Zellen bei 300xg and 4°C für 5 min wurden sie in Medium gelöst. Widerum wurden Aliquots für Steriltests, Tests zu Zellwiederherstellung u.- lebensfähigkeit, für FACS-Analysen, und zur Bestimmung der die Zytokin-Expression zurückbehalten. Die Transfektions-Effizienz wurde wie beschrieben bestimmt (Foa et al., 1994, Nat. Immun. 13: 65-75)).

Die transfizierte Tumorzellsuspension wurde abermals getrocknet, in 1,5 ml Gefriermedium aufgenommen (80% FCS, 10% DMSO, 10% Suspensionsmedium) und in Portionen zu Zellen von 8x10⁶ bis 1,4x107 in versiegelten 2 ml Gefriertubes aufbewahrt. Die Röhrchen wurden bei -80°C a 1°C/min in flüssigem Stickstoff gelagert.

### Komplettierung der erfindungsgemäßen Vakzine

Nach vorsichtigem Auftauen der Gefriertubes wurde nochmals die Zellzahl bestimmt und Aliquote für Sterilitätstests zurückbehalten. Nach sorgfältigem mehrmaligen Waschen der Zellen wurde das erhaltene Zellpellet in Dulbecco's Phosphate Buffered Saline (DPBS, Camprex Kat. Nr.:17-512F) resuspendiert. Je Ansatz wurden zu der Suspension 500 Mikrogramm des Adjuvants DSLIM hinzugefügt. Die komplettierte Vakzine wurde in ein steriles endotoxinfreies Reaktionsgefäß überführt. Jeweils 750 Mikroliter dieses Ansatzes wurden mittels Kanüle in Spritzen aufgezogen.

Vor der Applikation erfolgte die fünfmalige Bestrahlung der Spritzen mit Gamma-Strahlung, was einer Gesamtdosis von 150 Gray entsprach.

### Lymphozytenpräparation

Periphäre Blutmonozyten (PBMC) wurden aus heparinisierten periphären Blut mittels Lymphoprep (GIBCO, Karlsruhe) mittels Dichtezentrifugation erhalten. Die Zellen wurden zweimal mit PBS gewaschen und in flüssigem Stickstoff in der Anwesenheit von mehr als 20% FCS für ELISPOT und zytotoxische Assays gefriergetrocknet.

### Statistische Analyse

Wilcoxons angepaßter paarweiser und unpaarweiser Test wurde zur Analyse der statistischen Signifikants benutzt. Ein p- Wert < .0,05 wird als signifikant betrachtet.

## Patentansprüche

1. Vakzine zur Behandlung von Patienten mit Tumorerkrankungen, enthaltend allogene Tumorzellen und immunmodulierende Oligodesoxyribonukleotide als Adjuvanz, wobei die Tumorzellen zuvor ex-vivo mit für Interleukin-7 (IL-7), Granulozyten-Makrophagen-Koloniestimulierenden Faktor (GM-CSF), CD40L/CD154 und B7.1/CD80 kodierenden Nukleinsäuremolekülen transfiziert wurden.

2. Vakzine zur Behandlung von Patienten mit Tumorerkrankungen, enthaltend allogene Tumorzellen und immunmodulierende Oligodesoxyribonukleotide als Adjuvanz, wobei die Tumorzellen zuvor ex-vivo mit für Interleukin-7 (IL-7), Granulozyten-Makrophagen-Koloniestimulierenden Faktor (GM-CSF) und CD40L/CD154 kodierenden Nukleinsäuremolekülen transfiziert wurden.

3. Vakzine nach Anspruch 1 oder 2, wobei die kodierenden Nukleinsäuremoleküle in einem oder mehreren Expressionskonstrukten vorliegen.

4. Vakzine nach Anspruch 3, wobei das Expressionskonstrukt
a) ein Plasmid ist, oder
b) eine linear-doppelsträngige, kovalent geschlossene Expressionskassette ist, die lediglich aus einem CMV Promotor, einem Intron, der kodierenden Gensequenz und einer Polyadenylierungssequenz besteht, welche an beiden Enden des Doppelstrangs durch eine kurze Schleife einzelsträngiger Nukleotidreste kovalent geschlossen ist.

5. Vakzine nach einem oder mehreren der Ansprüche 1 bis 4, wobei das immunmodulierende Oligodesoxyribonukleotid
a) eine Sequenz mit der Basenfolge N¹N²CGN³N⁴ umfasst, wobei N¹N² ein Element ausgewählt aus der Gruppe umfassend GT, GG, GA, AT oder AA, N³N⁴ ein Element ausgewählt aus der Gruppe umfassend CT oder TT, sowie C Desoxycytosin, G Desoxyguanosin, A Desoxyadenosin und T Desoxythymidin ist,
b) und einen zirkulären Strang Desoxyribonukleinsäure mit einer teilweise zueinander komplementären, antiparallelen Basensequenz umfasst und hantelförmig ausgebildet ist.

6. Vakzine nach Anspruch 5, wobei die Sequenz mit der Basenfolge N¹N²CGN³N⁴ im einsträngigen Bereich des Oligodesoxyribonukleotids positioniert ist und 40 bis 200 Nukleotide umfasst.

7. Vakzine nach einem oder mehreren der Ansprüche 1 bis 6, wobei diese einen pharmazeutisch verträglichen Träger umfasst.

8. Vakzine nach Anspruch 1 oder 2, wobei die allogenen Tumorzellen ausgewählt sind aus kolorektalem Karzinom, kleinzelligem und nicht-kleinzelligem Lungenkarzinom, Prostatakarzinom, Mammakarzinom, Ovarialkarzinom und Nierenzellkarzinom, sowie malignem Melanom.

9. Vakzine nach einem oder mehreren der Ansprüche 1 bis 8, wobei die Tumorzellen aus der Nierenkarzinomzelllinie stammen, welche unter der Nummer DSM ACC 2635 bei der DSMZ hinterlegt ist.

10. Verfahren zur Herstellung einer Vakzine nach den Ansprüchen 1 bis 8 zur Behandlung von Patienten mit Tumorerkrankungen, wobei allogene Tumorzellen ex-vivo mit
a) für Interleukin-7 (IL-7), Granulozyten-Makrophagen-Koloniestimulierenden Faktor (GM-CSF), CD40L/CD154 und B7.1/CD80, oder
b) für Interleukin-7 (IL-7), Granulozyten-Makrophagen-Koloniestimulierenden Faktor (GM-CSF) und CD40L/CD154
kodierenden Nukleinsäuremolekülen transfiziert werden und anschließend immunmodulierende Oligodesoxyribonukleotide als Adjuvanz zugesetzt werden und abschließend die Überführung in eine applikationsfähige pharmazeutische Zusammensetzung erfolgt.

11. Verfahren nach Anspruch 10, wobei das immunmodulierende Oligodesoxyribonukleotid einen zirkulären Strang Desoxyribonukleinsäure mit einer teilweise zueinander komplementären, antiparallelen Basensequenz umfasst und hantelförmig ausgebildet ist.

12. Verfahren nach Anspruch 10 oder 11, wobei das immunmodulierende Oligodesoxyribonukleotid eine Sequenz mit der Basenfolge N¹N²CGN³N⁴ umfasst, wobei N¹N² ein Element ausgewählt aus der Gruppe umfassend GT, GG, GA, AT oder AA, N³N⁴ ein Element ausgewählt aus der Gruppe umfassend CT oder TT, sowie C Desoxycytosin, G Desoxyguanosin, A Desoxyadenosin und T Desoxythymidin ist.

13. Verfahren nach Anspruch 12, wobei die Sequenz mit der Basenfolge N¹N²CGN³N⁴ im einsträngigen Bereich des Oligodesoxyribonukleotids positioniert ist und 40 bis 200 Nukleotide umfasst.

14. Verfahren nach Anspruch 10, wobei die Nukleinsäuremoleküle in einem oder mehreren Expressionskonstrukten vorliegen.

15. Verfahren nach einem oder mehreren der Ansprüche 10 bis 14, **dadurch gekennzeichnet, dass** die Transfektion mittels ballistischem Transfer, Polykationen-Transfektion, Kalziumphosphatpräzipitation, Mikroinjektion, Protoplastenfusion, Liposomenfusion, virale Transfektionssysteme, Lipofektion und/oder Elektroporation erfolgt.

16. Verfahren nach Anspruch 10, wobei die allogenen Tumorzellen ausgewählt sind aus kolorektalem Karzinom, kleinzelligem und nicht-kleinzelligem Lungenkarzinom, Prostatakarzinom, Mammakarzinom, Ovarialkarzinom und Nierenzellkarzinom, sowie malignem Melanom.

17. Verfahren nach Anspruch 10, wobei die Tumorzellen von mehreren Patienten mit gleichem Krankheitsbild stammen.

18. Verfahren nach einem der Ansprüche 10 bis 17, wobei Tumorzellen der Nierenkarzinomzelllinie verwendet werden, welche unter der Nummer DSM ACC 2635 bei der DSMZ hinterlegt ist.

19. Humane Tumorzelle, die ex-vivo mit
a) für Interleukin-7 (IL-7), Granulozyten-Makrophagen-Koloniestimulierenden Faktor (GM-CSF), CD40UCD154 und B7.1/CD80, oder
b) für Interleukin-7 (IL-7), Granulozyten-Makrophagen-Koloniestimulierendern Faktor (GM-CSF) und CD40L/CD154
kodierenden Nukleinsäuremolekülen transfiziert wurde und entsprechende Expressionskonstrukte aufweist.

20. Humane Tumorzelle nach Anspruch 19, wobei das Expressionskonstrukt
a) ein Plasmid ist, oder
b) eine linear-doppelsträngige, kovalent geschlossene Expressionskassette ist, die lediglich aus einem CMV Promotor, einem Intron, der kodierenden Gensequenz und einer Polyadenylierungssequenz besteht, welche an beiden Enden des Doppelstrangs durch eine kurze Schleife einzelsträngiger Nukleotidreste kovalent geschlossen ist.

21. Humane Tumorzelle nach Anspruch 19 und 20 wobei es sich um eine allogene Tumorzelle einer Nierenzellkarzinomzelllinie handelt.

22. Humane Tumorzelle nach Anspruch 21, wobei es sich um Tumorzellen der Nierenzellkarzinomzelllinie handelt, welche unter der Nummer DSM ACC 2635 bei der DSMZ hinterlegt ist.

23. Humane Tumorzelle nach einem oder mehreren der Ansprüche 19 bis 22 zur Verwendung als Vakzine zur Behandlung von Tumorerkrankungen.

## Claims

1. Vaccine for the treatment of patients with tumour diseases, comprising allogeneic tumour cells and immunomodulating oligodeoxyribonucleotides as an adjuvant, wherein the tumour cells were transfected previously ex-vivo with nucleic acid molecules encoding interleukin-7 (IL-7), granulocyte-macrophage colony stimulating factor (GM-CSF), CD40UCD154 and B7.1/CD80.

2. Vaccine for the treatment of patients with tumour diseases, comprising allogeneic tumour cells and immunomodulating oligodeoxyribonucleotides as an adjuvant, wherein the tumour cells were transfected previously ex-vivo with nucleic acid molecules encoding interleukin-7 (IL-7), granulocyte-macrophage colony stimulating factor (GM-CSF) and CD40L/CD154.

3. Vaccine according to claim 1 or 2, wherein the encoding nucleic acid molecules are present in one or more expression constructs.

4. Vaccine according to claim 3, wherein the expression construct is
a) a plasmid or
b) a linear double stranded, covalently closed expression cassette consisting solely of a CMV promoter, an intron, the encoding gene sequence and a polyadenylation sequence being covalently closed on both ends of the double strand by a short loop of single stranded nucleotide residues.

5. Vaccine according to one or several of claims 1 to 4, wherein the immunomodulating oligodeoxyribonucleotide
a) comprises a sequence of the base sequence N¹N²CGN³N⁴, wherein N¹N² is an element selected from the group comprising GT, GG, GA, AT or AA, N³N⁴ is an element selected from the group comprising CT or TT, and C is deoxycytosine, G is deoxyguanosine, A is de-oxyadenosine and T is deoxythymidine,
b) and comprises a circular strand of deoxyribonucleic acid with a partially complementary, antiparallel base sequence and is of a dumbbell shape.

6. Vaccine according to claim 5, wherein the sequence with the base sequence N¹N²CGN³N⁴ is positioned in the single stranded region of the oligodeoxyribonucleotide and comprises 40 to 200 nucleotides.

7. Vaccine according to one or several of claims 1 to 6, wherein said vaccine comprises a pharmaceutically acceptable carrier.

8. Vaccine according to claim 1 or 2, wherein the allogeneic tumour cells are selected from colorectal carcinoma, small cell or non-small cell lung carcinoma, prostate carcinoma, mamma carcinoma, ovarian carcinoma, renal cell carcinoma and malign melanoma.

9. Vaccine according to one or several of claims 1 to 8, wherein the tumour cells are derived from the renal carcinoma cell line deposited under accession number DSM ACC 2635 at the DSMZ.

10. Method for the manufacture of a vaccine according to claims 1 to 8 for the treatment of patients with tumour diseases, wherein allogeneic tumour cells are transfected ex-vivo with nucleic acid molecules encoding
a) interleukin-7 (IL-7), granulocyte-macrophage colony stimulating factor (GM-CSF), CD40UCD154 and B7.1/CD80, or
b) interleukin-7 (IL-7), granulocyte-macrophage colony stimulating factor (GM-CSF), and CD40UCD154
and subsequently immunomodulating oligodeoxyribonucleotides are added as an adjuvant and finally the transfer into an applicable pharmaceutical composition is carried out.

11. Method according to claim 10, wherein the immunomodulating oligodeoxyribonucleotide comprises a circular strand of deoxyribonucleic acid with a partially complementary, antiparallel base sequence and is of a dumbbell shape.

12. Method according to claim 10 or 11, wherein the immunomodulating oligodeoxyribonucleotide comprises a sequence of the base sequence N¹N²CGN³N⁴, wherein N¹N² is an element selected from the group comprising GT, GG, GA, AT or AA, N³N⁴ is an element selected from the group comprising CT or TT, and C is deoxycytosine, G is deoxyguanosine, A is de-oxyadenosine and T is deoxythymidine.

13. Method according to claim 12, wherein the sequence with the base sequence N¹N²CGN³N⁴ is positioned in the single stranded region of the oligodeoxyribonucleotide and comprises 40 to 200 nucleotides.

14. Method according to claim 10, wherein the nucleic acid molecules are present in one or several expression constructs.

15. Method according to one or several of claims 10 to 14, **characterised by** the transfection method being ballistic transfer, polykation transfection, calcium-phosphate precipitation, microinjection, protoplast fusion, liposome fusion, viral transfection systems, lipofection and/ or electroporation.

16. Method according to claim 10, wherein the allogeneic tumour cells are selected from colorectal carcinoma, small cell or non-small cell lung carcinoma, prostate carcinoma, mamma carcinoma, ovarian carcinoma, renal cell carcinoma and malign melanoma.

17. Method according to claim 10, wherein the tumour cells are derived from several patients with the same disease picture.

18. Method according to one of the claims 10 to 17, wherein tumour cells of the renal carcinoma cell line deposited under accession number DSM ACC 2635 at the DSMZ are employed.

19. Human tumour cell transfected ex-vivo with nucleic acid molecules encoding
a) interleukin-7 (IL-7), granulocyte-macrophage colony stimulating factor (GM-CSF), CD40UCD154 and B7.1/CD80, or
b) interleukin-7 (IL-7), granulocyte-macrophage colony stimulating factor (GM-CSF), and CD40UCD154
and comprising the corresponding expression constructs.

20. Human tumour cell according to claim 19, wherein the expression construct is
a) a plasmid or
b) a linear double stranded, covalently closed expression cassette consisting solely of a CMV promoter, an intron, the encoding gene sequence and a polyadenylation sequence being covalently closed on both ends of the double strand by a short loop of single stranded nucleotide residues.

21. Human tumour cell according to claim 19 and 20, wherein said tumour cell is an allogeneic tumour cell of a renal cell carcinoma cell line.

22. Human tumour cell according to claim 21, wherein the tumour cells are the renal cell carcinoma cell line deposited under accession number DSM ACC 2635 at the DSMZ.

23. Human tumour cell according to one or several of claims 19 to 22 for the use as a vaccine for the treatment of tumour diseases.

## Revendications

1. Vaccin destiné au traitement de patients atteints de maladies tumorales, comprenant des cellules tumorales allogéniques et des oligodésoxyribonucléotides d'immunomodulation en tant qu'adjuvant, dans lequel les cellules tumorales sont transfectées au préalable ex vivo avec des molécules d'acides nucléiques codant l'interleukine-7 (IL-7), un facteur stimulant la formation et le développement de colonies de granulocytes - macrophages (GM-CSF), un CD40L/CD154 et un B7.1/CD80.

2. Vaccin destiné au traitement de patients atteints de maladies tumorales, comprenant des cellules tumorales allogéniques et des oligodésoxyribonucléotides d'immunomodulation en tant qu'adjuvant, dans lequel les cellules tumorales sont transfectées au préalable ex vivo avec des molécules d'acides nucléiques codant l'interleukine-7 (IL-7), un facteur stimulant la formation et le développement de colonies de granulocytes - macrophages (GM-CSF) et un CD40L/CD154.

3. Vaccin selon la revendication 1 ou 2, dans lequel les molécules d'acides nucléiques codantes sont présentes dans une ou plusieurs constructions d'expression.

4. Vaccin selon la revendication 3, dans lequel la construction d'expression est
a) un plasmide ou
b) une cassette d'expression fermée de façon covalente, à double brin linéaire, constituée uniquement d'un promoteur CMV, d'un intron, de la séquence de gène codante et d'une séquence de polyadénylation fermée de façon covalente sur les deux extrémités du double brin par une courte boucle de résidus de nucléotides à un seul brin.

5. Vaccin selon l'une ou plusieurs des revendications 1 à 4, dans lequel l'oligo-désoxyribonucléotide d'immunomodulation
a) comprend une séquence présentant la séquence de base N¹N²CGN³N⁴, dans lequel N¹N² est un élément sélectionné à partir du groupe comprenant GT, GG, GA, AT ou AA, N³N⁴ est un élément sélectionné à partir du groupe comprenant CT ou TT, et C est la désoxycytosine, G est la désoxyguanosine, A est la désoxyadénosine et T est la désoxythymidine,
b) et comprend un brin circulaire d'acide désoxyribonucléique présentant une séquence de base antiparallèle partiellement complémentaire et présente une forme en haltère.

6. Vaccin selon la revendication 5, dans lequel la séquence présentant la séquence de base N¹N²CGN³N⁴ est positionnée dans la région à un seul brin de l'oligo-désoxyribonucléotide et comprend de 40 à 200 nucléotides.

7. Vaccin selon l'une ou plusieurs des revendications 1 à 6, dans lequel ledit vaccin comprend un support pharmaceutiquement acceptable.

8. Vaccin selon la revendication 1 ou 2, dans lequel les cellules tumorales allogéniques sont sélectionnées à partir du groupe constitué d'un carcinome colorectal, d'un carcinome du poumon à petites cellules et d'un carcinome du poumon non à petites cellules, d'un carcinome de la prostate, d'un carcinome du sein, d'un carcinome des ovaires, d'un carcinome de cellule rénale et d'un mélanome malin.

9. Vaccin selon l'une ou plusieurs des revendications 1 à 8, dans lequel les cellules tumorales sont dérivées de la lignée cellulaire de carcinome rénal déposée sous le numéro d'entrée DSM ACC 2635 au niveau de la banque DSMZ.

10. Procédé de fabrication de vaccin selon les revendications 1 à 8, destiné au traitement de patients atteints de maladies tumorales, dans lequel les cellules tumorales allogéniques sont transfectées ex vivo avec des molécules d'acides nucléiques codant
a) l'interleukine-7 (IL-7), un facteur stimulant la formation et le développement de colonies de granulocytes - macrophages (GM-CSF), un CD40L/CD154 et un B7.1/CD80, ou
b) l'interleukine-7 (IL-7), un facteur stimulant la formation et le développement de colonies de granulocytes - macrophages (GM-CSF), et un CD40L/CD 154
et par la suite les oligodésoxyribonucléotides d'immunomodulation sont ajoutés en tant qu'adjuvant et pour finir le transfert dans une composition pharmaceutiquement acceptable est effectué.

11. Procédé selon la revendication 10, dans lequel l'oligodésoxyribonucléotide d'immunomodulation comprend un brin circulaire d'acide désoxyribonucléique présentant une séquence de base antiparallèle partiellement complémentaire et présente une forme en haltère.

12. Procédé selon la revendication 10 ou 11, dans lequel l'oligodésoxyribonucléotide d'immunomodulation comprend une séquence présentant la séquence de base N¹N²CGN³N⁴, dans lequel N¹N² est un élément sélectionné à partir du groupe comprenant GT, GG, GA, AT ou AA, N³N⁴ est un élément sélectionné à partir du groupe comprenant CT ou TT, et C est la désoxycytosine, G est la désoxyguanosine, A est la désoxyadénosine et T est la désoxythymidine.

13. Procédé selon la revendication 12, dans lequel la séquence présentant la séquence de base N¹N²CGN³N⁴ est positionnée dans la région à un seul brin de l'oligo-désoxyribonucléotide et comprend de 40 à 200 nucléotides.

14. Procédé selon la revendication 10, dans lequel les molécules d'acides nucléiques sont présentes dans une ou plusieurs constructions d'expression.

15. Procédé selon l'une ou plusieurs des revendications 10 à 14, **caractérisé par** la méthode de transfection qui est un transfert balistique, une transfection par polycations, une précipitation de phosphate de calcium, une micro-injection, une fusion de protoplastes, une fusion de liposomes, des systèmes de transfection virale, une lipofection et/ou une électroporation.

16. Procédé selon la revendication 10, dans lequel les cellules tumorales allogéniques sont sélectionnées à partir d'un carcinome colorectal, d'un carcinome du poumon à petites cellules et d'un carcinome du poumon non à petites cellules, d'un carcinome de prostate, d'un carcinome du sein, d'un carcinome des ovaires, d'un carcinome de cellule rénale et d'un mélanome malin.

17. Procédé selon la revendication 10, dans lequel les cellules tumorales sont dérivées de plusieurs patients présentant le même tableau clinique.

18. Procédé selon l'une des revendications 10 à 17, dans lequel les cellules tumorales de la lignée cellulaire de carcinome rénal déposée sous le numéro d'entrée DSM ACC 2635 au niveau de la banque DSMZ sont employées.

19. Cellule tumorale humaine transfectée ex vivo par des molécules d'acides nucléiques codant
a) l'interleukine-7 (IL-7), un facteur stimulant la formation et le développement de colonies de granulocytes - macrophages (GM-CSF), un CD40L/CD154 et un B7.1/CD80, ou
b) l'interleukine-7 (IL-7), un facteur stimulant la formation et le développement de colonies de granulocytes - macrophages (GM-CSF), et un CD40L/CD154 et comprenant les constructions d'expression correspondantes.

20. Cellule tumorale humaine selon la revendication 19, dans lequel la construction d'expression est
a) un plasmide ou
b) une cassette d'expression fermée de façon covalente, à double brin linéaire, constituée uniquement d'un promoteur CMV, d'un intron, de la séquence de gène codante et d'une séquence de polyadénylation fermée de façon covalente sur les deux extrémités du double brin par une courte boucle de résidus de nucléotides à un seul brin.

21. Cellule tumorale humaine selon la revendication 19 et la revendication 20, dans lequel ladite cellule tumorale est une cellule tumorale allogénique d'une lignée cellulaire de carcinome de cellules rénales.

22. Cellule tumorale humaine selon la revendication 21, dans lequel les cellules tumorales sont issues d'une lignée cellulaire de carcinome de cellules rénales déposée sous le numéro d"entrée DSM ACC 2635 au niveau de la banque DSMZ.

23. Cellule tumorale humaine selon l'une ou plusieurs des revendications 19 à 22, destinée à être utilisée en tant que vaccin destiné au traitement de maladies tumorales.
